# EUROPEAN PATENT APPLICATION

(11) **EP 3 000 910 A1**
(43) Date of publication of application: **30.03.2016**
(21) Application number: 13884286.9
(22) Date of filing: 10.05.2013
(51) Int. Cl.: C23C 8/20

(54) **SOFTWARE AND METHOD FOR CALCULATING CARBON CONCENTRATION DISTRIBUTION**

(71) Applicant: Hitachi, Ltd., Chiyoda-ku Tokyo 100-8280 (JP)
(72) Inventor: PARK Minseok, Tokyo 100-8280 (JP)
(74) Representative: Beetz & Partner mbB
(86) International application number: PCT/JP2013/063106
(87) International publication number: WO 2014/181453

(57) **Abstract**

A method of calculation of carbon concentration distribution in a member as a result of carburizing includes calculating n-dimensional carbon diffusion of the member, determining calculation region for which (n+1)-dimensional carbon diffusion of the member is calculated, determining a boundary condition of the (n+1)-dimensional calculation region, and calculating the (n+1)-dimensional carbon diffusion by applying the n-dimensional calculation result to the boundary condition of the (n+1)-dimensional calculation region. Further, software for calculating carbon concentration distribution in a member as a result of carburizing includes outputting a calculation result by calculating n-dimensional carbon diffusion of the member, and outputting a calculation result by calculating (n+1) -dimensional carbon diffusion of the member by applying the n-dimensional calculation result to a boundary condition of a calculation region for which the (n+1) -dimensional carbon diffusion of the member is calculated.

## Description

### Technical Field

The present invention relates to a method of calculation of carbon concentration distribution as a result of carburizing in steel products and software in which the method is implemented.

### Background Art

When carburizing large steel members such as a gear, shaft and bearing of a wind power generator, railway vehicle, construction machinery, ship, and the like, efficiency of carbon supply to the steel members can be improved by carburizing under the low pressure carburizing (vacuum carburizing) method that uses a low pressure gas compared to the gas carburizing method.

However, in the low pressure carburizing, compared to the gas carburizing, it is difficult to control carburizing temperature, carburizing time, carburizing gas concentration, and the like, and it takes an enormous amount of time to predict carburizing conditions.

For example, in PTL 1, a method of finding appropriate carburizing conditions avoids repeating a lot of preliminary carburizing and predicts that how results will be when carburizing with some preset carburizing conditions by simulation using a computer, and on the basis of the results, success or failure is decided about whether or not the set carburizing conditions provide desirable results. In particular, carbon concentration distribution in the low pressure carburizing is predicted by dividing the surface portion of the component of the carburizing target into cubic cells in the range of 0.1 to 50 µm and solving a one-dimensional diffusion equation.

### Citation List

### Patent Literature

PTL 1: JP 2008-208403 A

### Summary of Invention

### Technical Problem

However, the technique of PTL 1 is not sufficiently considered for calculation of portions in which excess carburizing tends to occur, such as an edge portion and a corner portion. The edge portion is formed at a portion where two surfaces intersect, and the corner portion is formed at a portion where three surfaces intersect. Since carbon is permeated and diffused into these portions from a plurality of surfaces, to predict excess carburizing, it is desirable to include not only diffusion in vertical direction to the surface but also diffusion in parallel direction.

An object of the present invention is to predict excess carburizing at the time of the low pressure carburizing.

### Solution to Problem

According to the present invention, in a method of calculation of carbon concentration distribution in a member as a result of carburizing, the method includes the steps of: calculating n-dimensional carbon diffusion of the member; determining a calculation region for which (n+1)-dimensional carbon diffusion of the member is calculated; determining a boundary condition of the (n+1)-dimensional calculation region; and calculating the (n+1)-dimensional carbon diffusion by applying the n-dimensional calculation result to the boundary condition of the (n+1)-dimensional calculation region.

Further, software for calculating carbon concentration distribution in a member as a result of carburizing includes outputting a calculation result by calculating n-dimensional carbon diffusion of the member, and outputting a calculation result by calculating (n+1) -dimensional carbon diffusion of the member by applying the n-dimensional calculation result to a boundary condition of a calculation region for which the (n+1) -dimensional carbon diffusion of the member is calculated.

### Advantageous Effect of Invention

According to the present invention, excess carburizing at the time of low pressure carburizing can be predicted.

### Brief Description of Drawings

[FIG. 1] FIG. 1 is an explanatory diagram illustrating an example of a large member to be carburized.
[FIG. 2] FIG. 2 is an explanatory diagram illustrating a relationship between carburizing depth and regions.
[FIGS. 3(a) to 3(c) FIGS. 3(a) to 3(c) are explanatory diagrams illustrating a method of calculation of carbon concentration distribution as a result of carburizing.
[FIG. 4] FIG. 4 is a graph illustrating an example of a one-dimensional calculation result used to a virtual boundary condition of two-dimensional calculation.
[FIG. 5] FIG. 5 is a flow diagram illustrating processing of software according to Example 1.
[FIG. 6] FIG. 6 is a flow diagram illustrating processing of software according to Example 2.
[FIG. 7] FIG. 7 is a flow diagram illustrating processing of software according to Example 3.

### Description of Embodiments

A calculation of carbon concentration distribution as a result of carburizing is possible in principle by solving a diffusion equation by giving an appropriate boundary condition of the surface. The boundary condition includes the Neumann boundary condition that gives carbon concentration, and the Dirichlet boundary condition that gives a gradient of carbon concentration.

In low pressure carburizing, while efficiency of carbon supply is high, controlling is difficult compared to conventional gas carburizing. In particular, the carbon concentration in the steel product becomes excessively high, and it is difficult to prevent "excess carburizing" in which brittle iron carbide (Fe₃C, hereinafter cementite) is separated in the form of mesh along the boundary surface of the crystal grain. The excess carburizing tends to occur at edge portions and corner portions of the member in which a ratio of surface area per unit volume is high.

Therefore, it is important to express separation of the cementite in prediction of the carbon concentration distribution in the low pressure carburizing. Since the cementite is produced in a range of about several hundreds of micrometers from the surface, an element of the size of several tens of micrometers is required to express the cementite. Like this, when a small element is required for the prediction, the number of required elements increases, so that calculation time of the carbon concentration distribution becomes long. Even when an element measurement in the depth direction to the surface is made to be small without changing an element measurement in the horizontal direction, shortening of the calculation time has a limit, and a practical method is required to calculate the carbon concentration distribution in a short time.

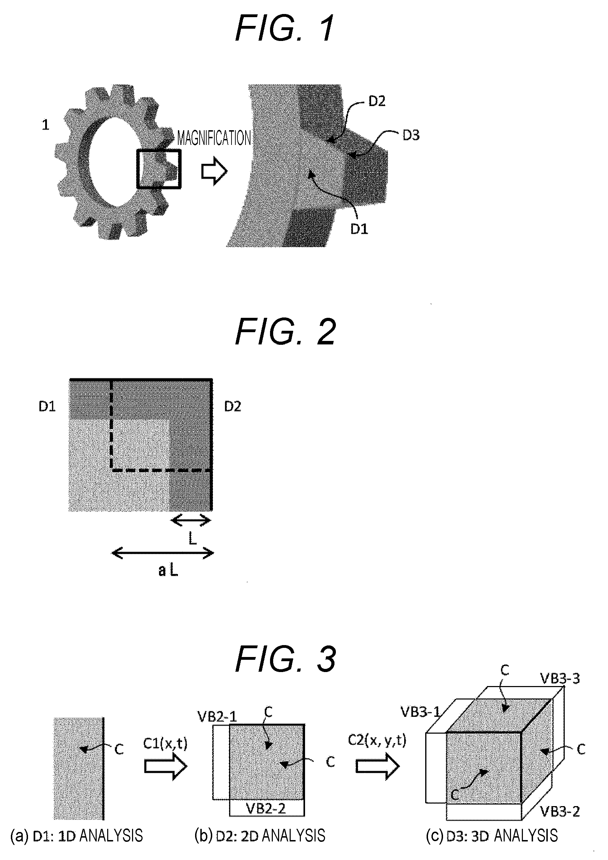

The present invention will be described referring to a gear illustrated in FIG. 1 as an example. Scope of the present invention is not limited to the gear, and the present invention is applicable to all steel products to be carburized, particularly large products. A gear 1 in FIG. 1 has a relatively complex shape including a number of edges and corners. In the gear 1, teeth having substantially the same shape are periodically connected together to constitute an entire gear. Analyzing one tooth in detail, the tooth surface can be decomposed into a planar region D1 that is away from the edge portion and the corner portion, a region D2 near the edge portion that is located at the edge portion and is away from the corner portion, and a region D3 near the corner portion.

The criterion for decomposing the region into D1, D2, D3 is set by using a carburizing depth L. The relationship between the carburizing depth L and the regions D1 and D2 is illustrated in FIG. 2. The carburizing depth L is a thickness of the layer in which the carbon concentration after carburizing is higher than the original carbon concentration of the base material, and can be estimated by solving a one-dimensional diffusion equation. The region D2 can be set to a distance of aL from the edge line. Here, a is a coefficient that simplifies two-dimensional diffusion calculation to one-dimensional diffusion calculation and considers variations in material and operating conditions, and is typically a value between 1 and 2.

The region D3 can be set to a distance of a'L from a corner point. Here, a' is a coefficient that simplifies three-dimensional diffusion calculation to one-dimensional diffusion calculation and considers variations in material and operating conditions, and is typically a value between 1 and 2. Since a' includes simplification of three-dimensional diffusion calculation to one-dimensional diffusion calculation, a' is greater than a. However, to omit troubles for independently setting a' and a, a value same as a can be used for a' in practical use.

A method of calculation of the present invention is particularly advantageous when a dimension of a product is significantly greater than the size of the regions D2, D3. For example, when a width of a tip of the tooth illustrated in FIG. 1 is greater than the size of the region D3, it is preferable to create a calculation model by dividing the tooth tip into the planar region D1, the region D2 near the edge, and the region D3 near the corner, instead of making the entire tooth tip be the calculation model. In this respect, the present invention is particularly effective for calculation of carbon concentration distribution when carburizing the large products with large dimensions.

FIG. 3 illustrates one-dimensional to three-dimensional methods of calculation of carbon concentration. First, the carbon concentration distribution is calculated in one dimension to the region D1, and a result C1(x, t) is obtained that represents space, time variation of the carbon concentration distribution (FIG. 3(a)). Since the region D1 is away from the edge portion and the corner portion, even one-dimensional calculation is sufficient in practical use. For a method of calculating carbon concentration distribution at the time of low pressure carburizing in one dimension, a number of presentations have been made in scientific journals and the like, and it is well known to those skilled in the art.

Since the region D1 has geometrically the same shape in any surfaces that can be approximated as a plane, typically only one region is calculated in the product. However, if there is a difference in carburizing temperature, material composition, and the surface state in the product, a plurality of regions can be calculated. On the other hand, in the region D2 and the region D3, since there are an angle between two surfaces intersecting at the edge (edge angle) and angles between three surfaces intersecting at the corner (corner angle) respectively, shapes are geometrically different. Accordingly, separate regions D2, regions D3 can be provided to a plurality of regions depending on a detailed shape of the product. Here, since the corner is a place where three edges gather, the corner angle is represented by a combination of three edge angles.

Next, carbon concentration distribution of the region D2 is calculated in two dimension by using C1(x, t) (FIG. 3(b)). The region D2 including the edge portion has four boundary lines. Two of the four boundary lines are surfaces through which carbon actually permeates, and the other two are virtual boundaries, VB2-1 and VB2-2, which are made between the region D1 and the region D2. The one-dimensional carbon diffusion result C1(x, t) is applied to the boundary conditions VB2-1 and VB2-2. When applying, if the element measurement of D1 matches the element measurement in the vertical direction from the surface of D2, C1(x, t) can be used as it is for the boundary conditions of VB2-1 and VB2-2. On the other hand, if the element measurement of D1 does not match the element measurement in the vertical direction from the surface of D2, C1(x, t) can be used for the boundary conditions of VB2-1 and VB2-2 by interpolating. If there is a difference in temperature, material composition, the surface state between VB2-1 and VB2-2, C1(x, t) can be calculated for each of VB2-1 and VB2-2 to be applied.

Next, carbon concentration distribution of the region D3 is calculated in three dimension by using a calculation result C2 (x, y, t) of the region D2 (FIG. 3(c)). The region D3 including the corner portion has six boundary surfaces. Three of the six boundary surfaces are surfaces through which carbon actually permeates, and the remaining three are virtual boundaries VB3-1, VB3-2, VB3-3, which are made between the region D2 and the region D3. C2(x, y, t) calculated in the region D2 of each corresponding edge angle is applied to VB3-1, VB3-2, VB3-3. When applying, as described above, C2(x, y, t) can be used as it is, and also can be used by interpolating.

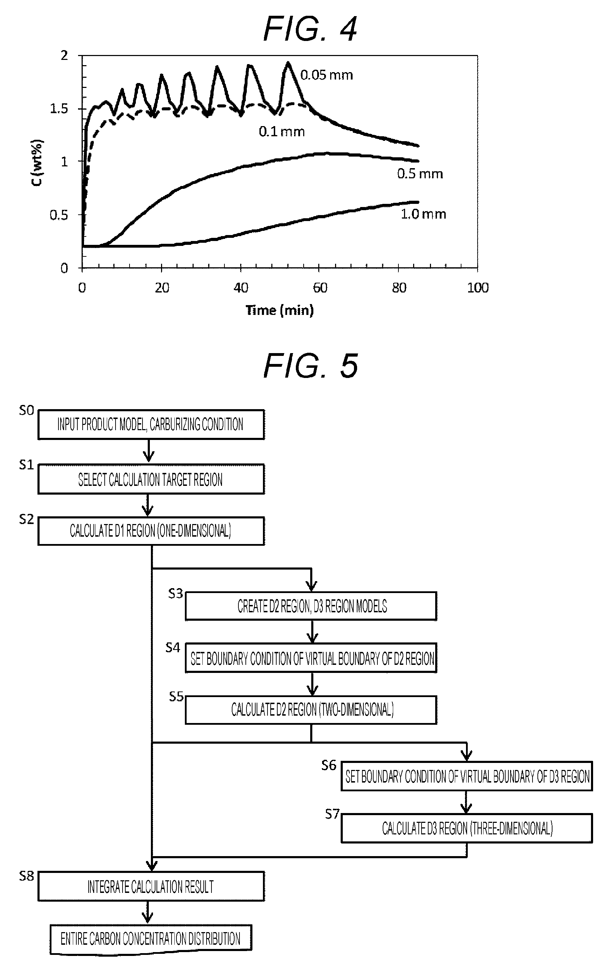

FIG. 4 illustrates an example of the one-dimensional calculation result C1(x, t) that is used for virtual boundary condition of two-dimensional calculation. The pulse carburizing method is used in which "carburizing stage" and "diffusion stage" are repeated, the carburizing stage introducing carburizing gas in a carburizing furnace, the diffusion stage exhausting the carburizing gas to perform only diffusion. FIG. 4 illustrates time variation of the carbon concentration at each point of depth from the surface of 0.05 mm, 0.1 mm, 0.5 mm, and 1 mm when 4% of carbon exists on the surface of the steel product in the carburizing stage. Although only the time variation of carbon concentration at four points is illustrated as representatives in FIG. 4, in practice the time variation of carbon concentration is given as the boundary condition for each point on the virtual boundary line in accordance with element division in the vertical direction from the surface of the region D2. Although only C1(x, t) is illustrated FIG. 4 for simplicity, C2 (x, y, t) is obtained in the same manner.

The method of calculation of the present invention, as described above, uses the calculation result of the region D1 for the boundary condition of the region D2, and uses the calculation result of the region D2 for the boundary condition of the region D3. That is, a calculation result of the immediately preceding dimension is used for a boundary condition of a calculation region determined in a dimension in which carbon concentration distribution is desired to obtain. Thus, only a part of the product can be the calculation model, so that the carbon concentration distribution can be calculated significantly faster than the conventional method of calculation in which the entire product is the calculation model. Further, in the cases of the edge portion and the corner portion that receive influence of diffusion from a plurality of surfaces, the diffusion in the parallel direction to the surface can be considered by performing two-dimensional calculation and three-dimensional calculation respectively, so that prediction of the excess carburizing is possible. On the basis of the prediction, the excess carburizing can be prevented also in actual carburizing.

Incidentally, although one-dimensional carbon diffusion and two-dimensional carbon diffusion are both calculated for three-dimensional calculation in the following examples, if the one-dimensional calculation result is originally prepared, the one-dimensional calculation can be omitted.

### Example 1

FIG. 5 illustrates an example of processing of software in which a method of calculation of carbon concentration distribution as a result of carburizing is implemented.
S0 is a step for inputting a product models and carburizing conditions. The product models include the shape of the product, chemical composition of the base material, and the like. The carburizing conditions include carburizing time, carburizing temperature, surface carbon concentration, and the like. A carbon permeation flow rate on the surface can be specified instead of the surface carbon concentration. The carburizing time includes both of the carburizing stage and the diffusion stage. If both of the carburizing stage and the diffusion stage are included, the surface carbon concentration or the carbon permeation rate is specified to each period.
S1 is a step for outputting a calculation target by analyzing the shape of the product. The corner angle, edge angle are analyzed as the shape of the product. If a plurality of portions exists that has the same corner angle and edge angle, all of the portions are considered as having the same carbon concentration distribution and only one of the portions is made to be the calculation target. Typically, only one portion is made to be the calculation target in the region D1. However, as described above, if there is distribution of chemical composition, the surface state, and temperature in the steel product, a plurality of portions may be made to be the calculation target.
S2 is a step for performing one-dimensional calculation in the region D1 of the calculation target output in S1 by using the carburizing conditions input in S0. The space, time variation C1(x, t) of the carbon concentration distribution in the vertical direction from the surface and the carburizing depth L are output.
S3 is a step for determining element division models (calculation region) of one or more of the region D2 and region D3 of the calculation target output in S1 by using the carburizing depth L that is the calculation result output in S2.
S4 is a step for applying C1(x, t) output in S2 to the boundary condition of the virtual boundary line of D2. When applying, as described above, C1(x, t) can be used as it is, and also can be used by interpolating.
S5 is a step for outputting C2(x, y, t) by performing two-dimensional calculation in each region D2 of the calculation target.
S6 is a step for applying C2(x, y, t) to the boundary condition of a virtual boundary surface of D3. As described above, one region D3 has respective three virtual boundary surfaces, and C2(x, y, t) calculated in the region D2 of the corresponding edge angle is applied to each virtual boundary surface. When applying, as described above, C2(x, y, t) can be used as it is, and also can be used by interpolating.
S7 is a step for outputting C3(x, y, z, t) by performing three-dimensional calculation in each calculation target of the region D3.
S8 is a step for outputting the carbon concentration distribution in the entire product surface by integrating C1 (x, t), C2(x, y, t), C3(x, y, z, t).

### Example 2

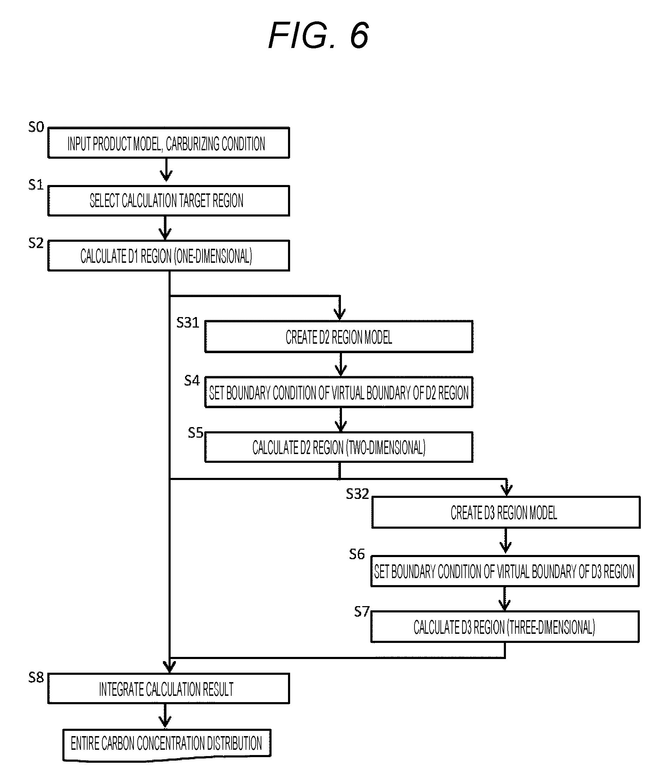

FIG. 6 illustrates another example of the processing of the software. Although models of the region D2 and the region D3 are created together in the step S3 in Example 1, the model of the region D2 is created in a step S31 after the step S2 and the model of the region D3 is created in a step S32 after the step S5 in this example.

At this time, in a step S32, a two-dimensional carburizing depth L' is obtained from C2(x, y, t) of a two-dimensional diffusion analysis result output by the step S5, and a region D3 model is created with a size a 'L'. The other steps are the same as those in Example 1. In this example, complexity of the software is increased greater than that of Example 1, but instead there is an advantage that the size of the region D3 can be decided with higher accuracy compared to Example 1 in which the one-dimensional carburizing depth L is used.

### Example 3

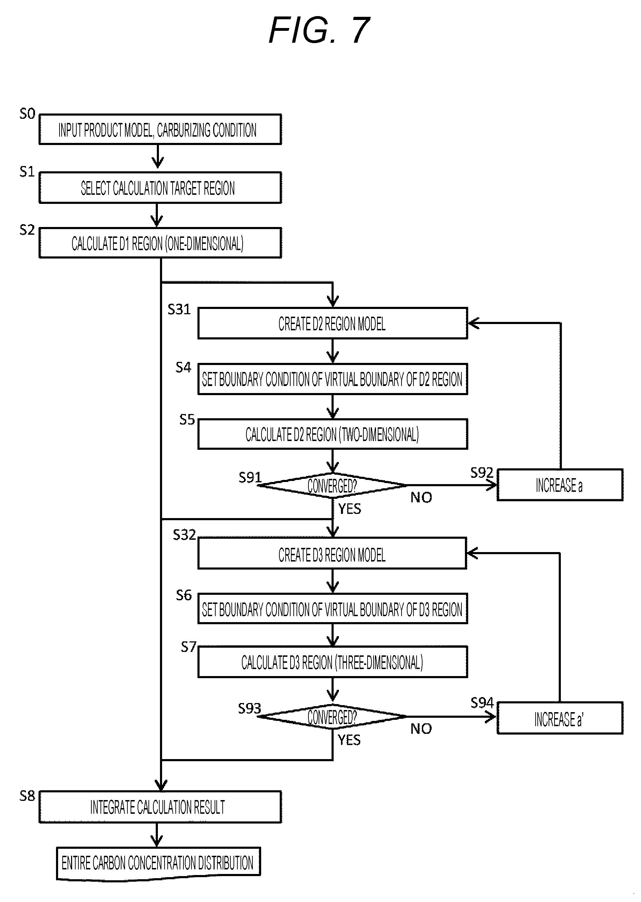

FIG. 7 illustrates another example of the processing of the software. This example is provided with a step S91 for deciding convergence of the calculation result C2 (x, y, t) with the size aL of the region D2, a step S92 for increasing a by a preset increment Δa when the calculation result is not converged in the step S91, a step S93 for deciding convergence of the calculation result C3(x, y, z, t) with the size a'L' or a'L of the region D3, and a step S94 for increasing a' by a preset increment Δa' when the calculation result is not converged in the step S93.

In the convergence decision in the steps S91 and S93, it is decided that the calculation is converged if a difference between the calculation result with smaller a, a' and the calculation result with larger a, a' is within a convergence criterion error determined by a user, and it is decided that the calculation is not converged if the difference is outside the convergence criterion error. In the steps S92 and S94, a, a' are increased and fed back to the steps S31 and S32 respectively, and the models of the region D2 and the region D3 are re-created. Incidentally, although feedback steps of S91-S94 are added to Example 2 in which the step S31 and the step S32 are separately provided in this example, the feedback steps can be added to Example 1 in which the region D2 model and the region D3 model are created together in the step S3, as well.

In this example, complexity of the software is increased greater than those of Example 1 and Example 2, but instead there is an advantage that size of the regions D2 and D3 can be determined with higher accuracy.

### Reference Signs List

- 1: gear
- D1: planar region
- D2: region near the edge portion
- D3: region near the corner portion
- VB: virtual boundary

## Claims

1. A method of calculation of carbon concentration distribution in a member as a result of carburizing, comprising the steps of:
calculating n-dimensional carbon diffusion of the member;
determining a calculation region for which (n+1) -dimensional carbon diffusion of the member is calculated;
determining a boundary condition of the (n+1)-dimensional calculation region; and
calculating the (n+1)-dimensional carbon diffusion by applying the n-dimensional calculation result to the boundary condition of the (n+1)-dimensional calculation region.

2. The method of calculation of claim 1, wherein n is 1.

3. The method of calculation of claim 1, wherein n is 2.

4. The method of calculation of claim 3, wherein
calculating two-dimensional carbon diffusion, where n is 2, further comprises the steps of:
calculating one-dimensional carbon diffusion of the member;
determining a calculation region for which two-dimensional carbon diffusion of the member is calculated;
determining a boundary condition of the two-dimensional calculation region; and
calculating the two-dimensional carbon diffusion by applying the n-dimensional calculation result to the boundary condition of the two-dimensional calculation region.

5. Software for calculating carbon concentration distribution in a member as a result of carburizing, the software comprising:
outputting a calculation result by calculating n-dimensional carbon diffusion of the member; and
outputting a calculation result by calculating (n+1)-dimensional carbon diffusion of the member by applying the n-dimensional calculation result to a boundary condition of a calculation region for which the (n+1)-dimensional carbon diffusion of the member is calculated.

6. The software of claim 5, wherein n is 1.

7. The software of claim 5, wherein n is 2.

8. The software of claim 6, further comprising
outputting a calculation result by calculating three-dimensional carbon diffusion of the member by applying the two-dimensional calculation result to a boundary condition of a calculation region for which the three-dimensional carbon diffusion of the member is calculated.
